# EUROPÄISCHE PATENTSCHRIFT

(11) **EP 2 699 204 B1**
(45) Veröffentlichungstag und Bekanntmachung des Hinweises auf die Patenterteilung: **19.08.2015**
(21) Anmeldenummer: 12706465.7
(22) Anmeldetag: 22.02.2012
(51) Int. Cl.: A61F 2/52

(54) **TEXTILER STOFF FÜR BEKLEIDUNGSSTÜCKE UND/ODER ORTHOPÄDISCHE ARTIKEL SOWIE KÜNSTLICHES BRUSTGEWEBETEIL MIT EINEM SOLCHEN STOFF**
TEXTILE FABRIC FOR ITEMS OF CLOTHING AND/OR ORTHOPAEDIC ARTICLES, AND ARTIFICIAL BREAST TISSUE PART COMPRISING SUCH A FABRIC
ÉTOFFE TEXTILE POUR DES ARTICLES D'HABILLEMENT ET/OU DES ARTICLES ORTHOPÉDIQUES AINSI QUE PIÈCE DE TISSU DE SEIN ARTIFICIELLE COMPRENANT UNE TELLE ÉTOFFE

(30) Priorität: 23.02.2011 DE 102011012111; 14.07.2011 DE 102011079147
(43) Veröffentlichungstag der Anmeldung: 26.02.2014
(73) Patentinhaber: Edelweiss Basics GmbH & Co. KG, 83098 Brannenburg (DE)
(72) Erfinder: WEBER-UNGER, Georg, A-6330 Kufstein (AT)
(74) Vertreter: Kador & Partner
(86) Internationale Anmeldenummer: PCT/EP2012/000770
(87) Internationale Veröffentlichungsnummer: WO 2012/113552

(56) Entgegenhaltungen:
- EP-A2- 0 688 547
- DE-U- 1 739 612
- US-A1- 2008 229 487

## Beschreibung

Die Erfindung betrifft einen textilen Stoff für die Produktion von Bekleidungsstücken und/oder orthopädischen Artikeln wie künstliche Brustgewebeteile insbesondere textile Stofflagen gemäß Anspruch 1.

Die Erfindung betrifft auch ein künstliches Brustgewebeteil zum Tragen in der Büstenschale eines Büstenschalen aufweisenden Kleidungsstücks gemäß Anspruch 7. Ein solches Brustgewebeteil wird im Folgenden als künstliches Brustgewebeteil der eingangs genannten Art bezeichnet.

Beispiele derartiger künstlicher Brustgewebeteile sind in Form von Brustprothesen aus der EP 0 688 547 A2, der EP 0 808 615 A2 und aus der EP 0 982 012 A2 bekannt. Während die aus der EP 0 688 547 A2 und der EP 0 808 615 A2 bekannten Brustprothesen eine Stofflage nur auf der Rückseite der jeweiligen Prothese aufweisen, hat die aus der EP 0 982 012 A2 bekannte Brustprothese eine vollständige Stoffummantelung.

Die aus diesen Druckschriften bekannten Brustprothesen sind dank der die Rückseite der Prothese bildenden luftdurchlässigen Stofflage und der von der Stofflage überspannten Vertiefung in der Rückseite des Brustprothesenkörpers sehr gut dafür geeignet, den von der Brustprothese abgedeckten Narbenbereich der Prothesenträgerin zu belüften und einer übermäßigen Schweißbildung in diesem Bereich entgegenzuwirken, die zu Staunässe führen könnte, die die Trägerin als unangenehm kühl empfinden würde. Manche Prothesenträgerinnen neigen jedoch zu so starker Schweißbildung, dass die mit den bekannten Brustprothesen erzielte Belüftung des Narbenbereichs nicht ausreichend ist, um ein Feuchtwerden der die Rückseite der Prothese bildenden Stofflage zu verhindern, wodurch der an sich sehr gute Tragekomfort dieser Brustprothesen zumindest temporär, beispielsweise bei stark schweißtreibender sportlicher Aktivität der Prothesenträgerin, beeinträchtigt werden kann.

Aus der EP 0 688 547 A2 ist ferner eine Brustprothese bekannt, die auf der Rückseite des Prothesenkörpers mehrere durch eine oder mehrere Versteifungsrippen voneinander getrennte Vertiefungen aufweist, die von der Stofflage überspannt werden, wobei die Stofflage auf der oder den Versteifungsrippen anliegen kann, wodurch die Durchströmung der Stofflage an den Stellen, an denen sie auf der oder den Versteifungsrippen anliegt, behindert werden kann.

Eine Brustprothese, bei der mehrere auf der Rückseite des Prothesenkörpers angeordnete Versteifungsrippen vorgesehen sind, die mehrere seitlich von ihnen angeordnete Vertiefungen in der Rückseite des Prothesenkörpers begrenzen, ist aus der EP 0 657 148 A1 bekannt. Durch Anbringen einer luftdurchlässigen Stofflage an der Rückseite der aus der EP 0 657 148 A1 bekannten Prothese entsteht eine Brustprothese, wie sie aus der EP 0 688 547 A2 bekannt ist.

US2008/229487 A1 zeigt eine textile Stofflage gemäß dem Oberbegriff von Anspruch 1.

Ausgehend von dem oben geschilderten Stand der Technik hat sich der Erfinder die Aufgabe gestellt, ein künstliches Brustgewebeteil der eingangs genannten Art so weiterzubilden, dass es noch bessere Belüftungseigenschaften als die bekannten Brustprothesen aufweist und sich durch einen mindestens so guten Tragekomfort, wie ihn die bekannten Brustprothesen bieten, auszeichnet.

Die Aufgabe der Erfindung wird dadurch gelöst, dass die textile Stofflage zumindest in dem über die Vertiefung sich erstreckenden Bereich eine Vielzahl von im Abstand voneinander angeordneten Belüftungslöchern aufweist, zwischen denen die Stofflage luftdurchlässig ist, so dass die Belüftungslöcher einen zusätzlichen Luftaustausch zwischen der Vertiefung und der äußeren Umgebung des Brustgewebeteils gestatten.

Da die Dichte und die Größe der Belüftungslöcher innerhalb weiter Grenzen variabel sind, kann die auf die gesamte Fläche der Stofflage bezogene Luftdurchlässigkeit mehr oder weniger stark über die in den keine Belüftungslöcher aufweisenden Bereichen der Stofflage herrschende Luftdurchlässigkeit hinaus erhöht werden, so dass sich der Vorteil ergibt, dass das erfindungsgemäße Brustgewebeteil hinsichtlich seiner Belüftungseigenschaften unabhängig von dem für die Stofflage gewählten Material an verschiedene Anwendungsfälle angepasst werden kann. Somit lässt sich das erfindungsgemäße Brustgewebeteil durch eine entsprechende Wahl der Dichte und/oder der Größe der Belüftungslöcher leicht mit einem stärkeren oder einem schwächeren Belüftungsvermögen bereitstellen, ohne dass das bisher bei den bekannten Brustprothesen verwendete Material für die Stofflage ausgetauscht werden müsste.

Die Stofflage weist zumindest im über der Vertiefung sich erstreckenden Bereich auf ihrer von der Rückseite des Körpers abgewandten Seite eine Vielzahl von im Abstand voneinander angeordneten Erhebungen auf, zwischen denen sich Senken befinden, von denen wenigstens einige mit Belüftungslöchern versehen sind. Die Erhebungen können linien-, punkt- und/oder flächenförmige Gebilde sein, zwischen denen sich die Senken befinden, die an der Oberseite offene Strömungskanäle bilden, die mit den Belüftungslöchern und der äußeren Umgebung des Brustgewebeteils in Strömungsverbindung sind. Die Erhebungen dienen somit als Abstandshalter, die zumindest die Ränder der Belüftungslöcher vom Oberkörper der Trägerin des Brustgewebeteils oder von einer eventuell zwischen dem Oberkörper der Trägerin und dem Brustgewebeteil befindlichen weiteren Stofflage, die beispielsweise Teil einer Prothesentasche sein kann, fern hält, so dass die Luft im Wesentlichen ungehindert in die Belüftungslöcher ein- und aus ihnen ausströmen kann. Die Belüftung wird durch die beim Tragen des Brustgewebeteils anstehenden Schwingungen und die daraus resultierende Pump- und Saugwirkung begünstigt.

Bei einem Ausführungsbeispiel können die Erhebungen aus durch Senken seitlich voneinander beabstandeten länglichen Rippen bestehen, die sich in Längsrichtung mit oder ohne Unterbrechungen zwischen der die Stofflage mit dem Körper verbindenden Naht erstrecken.

In diesem Fall sind die Belüftungslöcher im Abstand voneinander in den Senken zwischen den Rippen angeordnet. Um eine besonders günstige Be- und Entlüftung des von dem Brustgewebeteil abgedeckten Bereichs des Oberkörpers der Trägerin zu gewährleisten, können die Rippen geradlinig und parallel und im Tragezustand senkrecht verlaufen.

Bei einem anderen Ausführungsbeispiel bestehen die Erhebungen aus durch Senken seitlich voneinander beabstandeten Noppen, die im Bereich innerhalb der die Stofflage mit dem Körper verbindenden Naht verteilt angeordnet sind.

Eine besonders gute Belüftung wird bei einem Ausführungsbeispiel erzielt, bei dem die Erhebungen mehrere durch Senken seitlich voneinander beabstandete Gruppen von jeweils mehreren ringförmig um ein Belüftungsloch herum angeordneten Noppen aufweisen. Die Noppen jeder Gruppe sind hierbei durch Senken, die an der Oberseite offene Strömungskanäle bilden, die mit dem jeweiligen Belüftungsloch und den zwischen den Noppengruppen angeordneten Senken in Strömungsverbindung stehen, seitlich voneinander beabstandet. Die ringförmig um jeweils ein Belüftungsloch herum angeordneten Noppen verhindern zuverlässig, dass der Rand dieses Belüftungslochs in Kontakt mit dem Oberkörper der Trägerin oder einer eventuell zwischen dem Brustgewebeteil und dem Oberkörper der Trägerin befindlichen weiteren Stofflage, die beispielsweise Teil einer Prothesentasche sein kann, kommt. Die aus einem Belüftungsloch austretende Luft kann dann ungehindert in die Senken zwischen den ringförmig um das jeweilige Belüftungsloch angeordneten Noppen abströmen. Umgekehrt kann Luft ungehindert in die Belüftungslöcher einströmen. Vorzugsweise können die Erhebungen jeweils durch einen eine Flockbeschichtung tragenden Klebstoffauftrag auf der textilen Stofflage gebildet sein. Ein für die Bildung derartiger Erhebungen geeignetes Verfahren ist beispielsweise aus der DE 199 42 996 A1 bekannt.

Vorzugsweise erstreckt sich der die Belüftungslöcher und gegebenenfalls die Erhebungen aufweisende Bereich der textilen Stofflage nicht in die Schweißnaht, der die textile Stofflage mit dem Körper des Brustgewebeteils verbindet. Dadurch werden Inhomogenitäten der Schweißnaht vermieden.

Das erfindungsgemäße künstliche Brustgewebeteil eignet sich als Brustprothese oder Brustgewebeausgleichsteil für Frauen mit voll- bzw. teilamputierter Brust. Es ist aber auch als ein die natürliche Brust stützendes und/oder vergrößerndes Formteil verwendbar. Das erfindungsgemäße künstliche Brustgewebeteil eignet sich somit für medizinische und kosmetische Zwecke.

Erfindungsgemäß eignet sich der für die Stofflage verwendete textile Stoff auch für die Produktion von Bekleidungsstücken insbesondere Unterwäsche, Miederwaren wie BH's, Sport-BH's und Funktionswäsche, sowie anderen orthopädischen Artikeln wie Bandagen und Leibgurte.

Erfindungsgemäß hat der für die vorgenannten Verwendungszwecke geeignete Stoff somit eine Vielzahl von im Abstand voneinander angeordneten Erhebungen, zwischen denen sich Senken befinden, und befinden sich Belüftungslöcher in wenigstens einigen der Senken, wobei der Stoff zwischen den Belüftungslöchern auch luftdurchlässig ist.

Vorzugsweise sind die Erhebungen linien-, punkt- und/oder flächenförmige Gebilde, zwischen denen sich die Senken befinden, die an der Oberseite offene Strömungskanäle bilden, die mit den Belüftungslöchern in Strömungsverbindung sind.

Die Erhebungen können aus durch Senken seitlich voneinander beabstandete länglichen Rippen oder Noppen bestehen.

Alternativ können die Erhebungen mehrere Gruppen von jeweils mehreren ringförmig um ein Belüftungsloch herum angeordneten Noppen aufweisen, und können die Noppen der Gruppe durch Senken seitlich voneinander beabstandet sein, wobei diese Senken an der Oberseite offene Strömungskanäle bilden, die mit dem jeweiligen Belüftungsloch und den zwischen den Noppengruppen angeordneten Senken in Strömungsverbindung stehen.

Die Erhebungen können jeweils durch einen eine Flockenschichtung tragenden Klebstoffauftrag auf dem Stoff gebildet sein.

Alternativ können die Erhebungen durch Stricken oder Wirken gebildet sein.

Weitere vorteilhafte Eigenschaften des erfindungsgemäßen künstlichen Brustgewebeteils und des erfindungsgemäßen textilen Stoffes ergeben sich aus der folgenden Beschreibung verschiedener Ausführungsbeispiele der Erfindung in Verbindung mit den beiliegenden Zeichnungen. In den Zeichnungen zeigen:
- Fig. 1: eine Draufsicht auf die Rückseite einer gemäß einem Ausführungsbeispiel der Erfindung ausgebildeten Brustprothese, wobei ein Teil der rückseitigen textilen Stofflage weggebrochen ist, um die darunterliegende Struktur der Rückseite des Prothesenkörpers zu zeigen;
- Fig. 2: einen Querschnitt entlang der Linie II-II in Fig. 1, wobei die in Fig. 1 nur teilweise dargestellter Stofflage entlang der gesamten Linie II-II geschnitten dargestellt ist; und
- die Fig. 3A, 3B und 3C: jeweils eine Draufsicht auf verschiedene Ausführungsformen der textilen Stofflage, wobei die in Fig. 3B dargestellte Stofflage die gleiche ist, die auch in den Figuren 1 und 2 dargestellt ist.

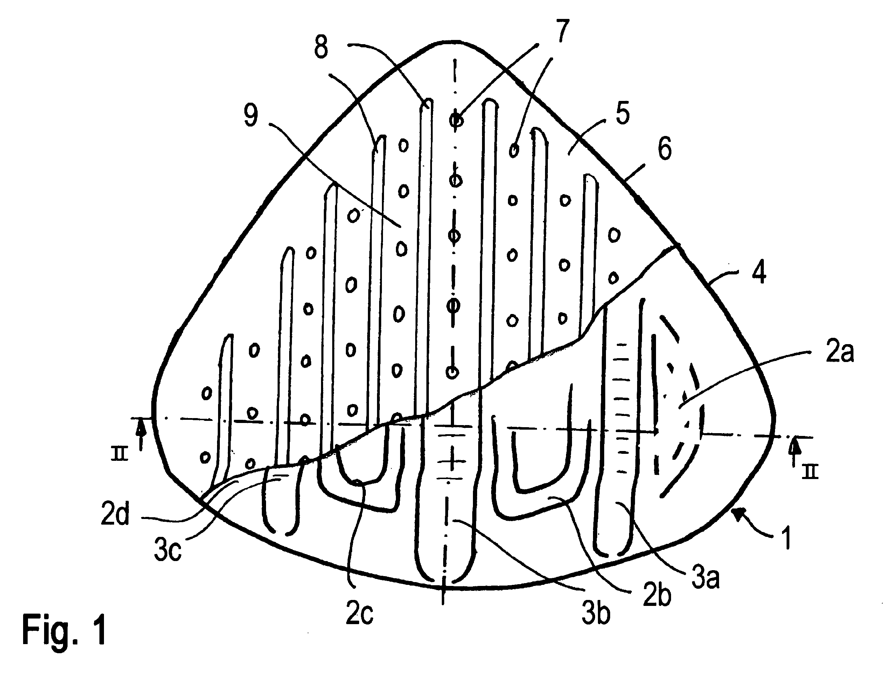

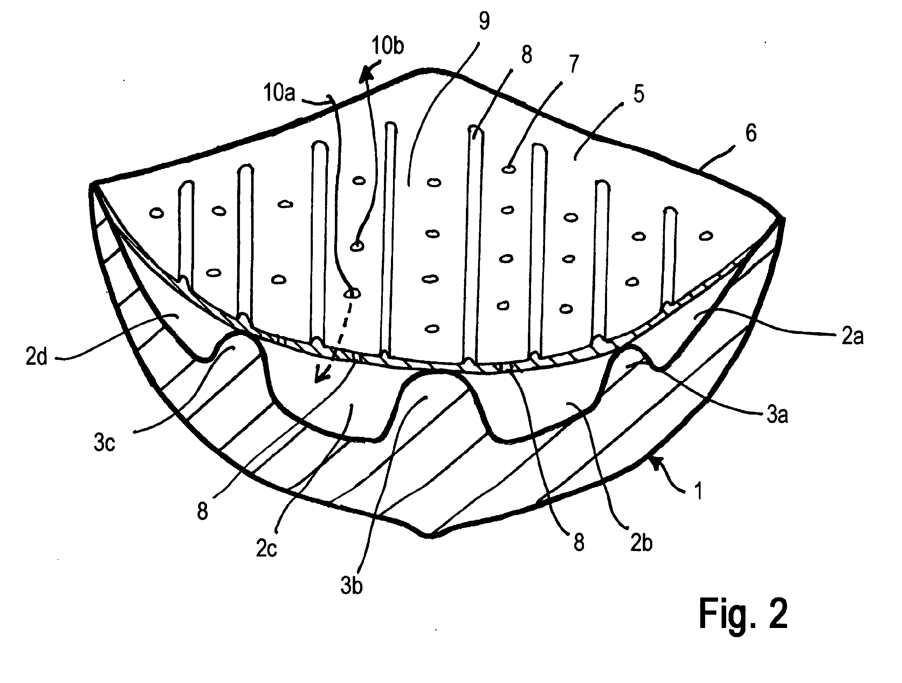

Wie aus den Figuren 1 und 2 ersichtlich ist, weist eine Brustprothese als Beispiel eines erfindungsgemäßen künstlichen Brustgewebeteils einen Prothesenkörper 1 auf, der eine Vertiefungen 2a, 2b, 2c und 2d sowie drei parallele, im Tragezustand senkrecht verlaufende Versteifungsrippen 3a, 3b und 3c aufweisende Rückseite hat. Der Prothesenkörper 1 weist einen weich-elastischen Kern, der beispielsweise aus einer additionsvernetzten Zwei-Komponenten-Silikonkautschukmasse besteht, und zwei längs einer eine geschlossene Schleife bildenden Naht 4 miteinander verschweißten Kunststofffolien, die beispielsweise Polyurethanfolien sind, auf. Die miteinander verschweißten Kunststofffolien bilden eine Hülle, in die die Silikonmasse hohlraumfrei eingeschlossen ist. Die Brustprothese weist außerdem eine textile Stofflage 5 auf, die an ihrem umlaufenden Rand 6 mit den Kunststofffolien an der Naht 4 verschweißt ist. Die Schweißnaht 4 der Kunststofffolien und der verschweißte Rand 6 der Stofflage 5 stellen somit eine gemeinsame Schweißnaht dar, die zugleich den seitlichen Rand der Prothese bildet. Die Stofflage 5 erstreckt sich über die gesamte Rückseite des Prothesenkörpers 1, so dass sie die Vertiefungen 2a bis 2d überspannt und an den Versteifungsrippen 3a bis 3c zumindest teilweise lose anliegt. An den Stellen, an denen die Stofflage 5 nicht an den Versteifungsrippen 3a bis 3c lose anliegt, erstreckt sie sich in einem geringen Abstand über die Versteifungsrippen hinweg. Die Vorderseite der hier beispielhaft dargestellten Brustprothese wird durch die Oberfläche der vorderen Kunststofffolie gebildet. Bei einem anderen, nicht dargestellten Ausführungsbeispiel könnte die Brustprothese auch auf der Vorderseite mit einer Stofflage versehen sein.

Die auf der Rückseite der Brustprothese angeordnete Stofflage 5 ist mit einer Vielzahl von Belüftungslöchern 7 versehen, die sich in einem Bereich befinden, der einen Abstand von dem Rand 6 der Stofflage 5 von einigen Millimetern hat. Innerhalb dieses Bereiches der Stofflage befindet sich auch eine Vielzahl von im Tragezustand der Prothese senkrecht verlaufenden Rippen 8, die Erhebungen darstellen. Zwischen jeweils zwei benachbarten Rippen 8 befindet sich eine Senke 9, in denen die Belüftungslöcher 7 angeordnet sind. Wie aus Fig. 2 ersichtlich ist, befinden sich die Rippen 8 nur auf der Seite der Stofflage 5, die von der Rückseite des Prothesenkörpers 1 abgewandt ist. Anders ausgedrückt, befinden sich die Rippen 8 nur auf derjenigen Seite der Stofflage 5, die im Tragezustand der Prothese dem Oberkörper der Prothesenträgerin zugewandt ist. Die Senken 9 zwischen den Rippen 8 bilden an der Oberseite offene Strömungskanäle, die mit den Belüftungslöchern 7 und der äußeren Umgebung der Brustprothese in Strömungsverbindung stehen. Die Luft kann somit aus den Vertiefungen 2a bis 2d durch die Belüftungslöcher 7 in die Senken 9 zwischen den Rippen 8 einströmen und von dort in die äußere Umgebung der Brustprothese abströmen, wie beispielsweise durch den Pfeil 10b in Fig. 2 symbolisiert ist. Umgekehrt kann Luft aus der äußeren Umgebung der Brustprothese über die Senken 9 in die Belüftungslöcher 7 und von dort in die Vertiefungen 2a bis 2d einströmen, wie durch den Pfeil 10a in Fig. 2 symbolisiert ist. Im Bereich zwischen den Belüftungslöchern 7 ist die Stofflage 5 wie bisher, luftdurchlässig. Die Belüftungslöcher 7 gestatten somit einen zusätzlichen Luftaustausch zwischen der äußeren Umgebung der Brustprothese und den Vertiefungen 2a bis 2d. Durch diesen Luftaustausch wird der Narbenbereich der Trägerin der Prothese, der von der Prothese abgedeckt wird, be- oder entlüftet. Die Erhebungen darstellenden Rippen 8 sorgen dafür, dass die Belüftungslöcher 7 nicht durch Anlage an dem Oberkörper der Trägerin oder an eine weitere Stofflage, die sich eventuell zwischen dem Oberkörper der Trägerin und der Brustprothese befindet, verschlossen werden können. Die weitere Stofflage kann zum Bespiel Teil einer Prothesentasche sein, in die die Prothese eingelegt ist, während sie in der Büstenschale eines Prothesenbüstenhalters getragen wird.

Die Rippen 8 können durch Stricken oder Wirken hergestellt werden, sie können aber auch durch einen Klebauftrag mit anschließender Beflockung mit Textilfasern hergestellt werden.

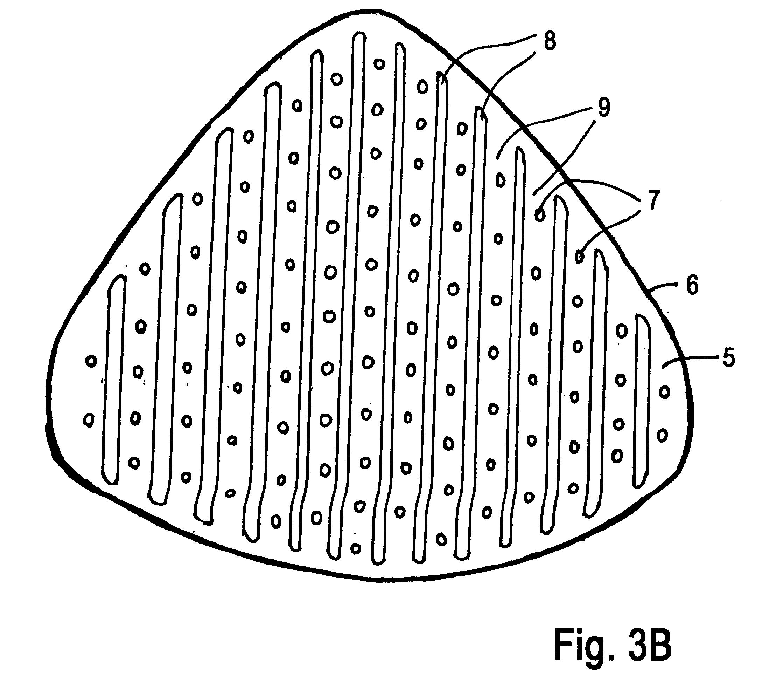

Fig. 3B zeigt die gesamte rückseitige textile Stofflage 5, die in den Figuren 1 und 2 nur teilweise bzw. geschnitten dargestellt ist.

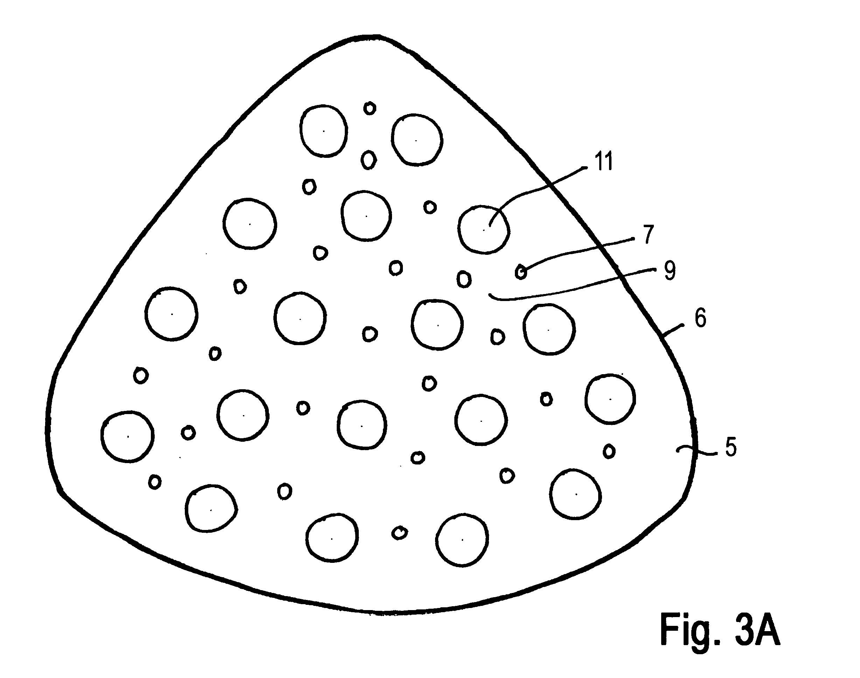

Bei einem anderen Ausführungsbeispiel, das in Fig. 3A dargestellt ist, bestehen die Erhebungen aus Noppen 11, die zwischen sich Senken 9 begrenzen, in denen Belüftungslöcher 7 angeordnet sind. Die Noppen 11 können durch einen Klebstoffauftrag mit anschließender Beflockung mit Textilfasern hergestellt werden. Auch bei diesem Ausführungsbeispiel sind die Senken 9 an der Oberseite offene Strömungskanäle, die mit den Belüftungslöchern 7 und der äußeren Umgebung der Brustprothese in Strömungsverbindung stehen.

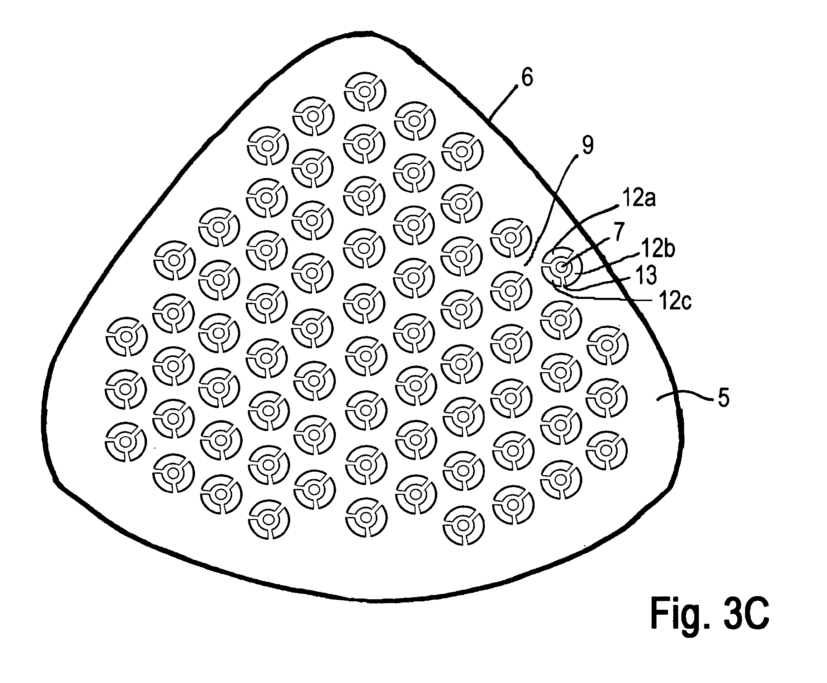

Bei einem noch weiteren Ausführungsbeispiel, das in Fig. 3C dargestellt ist, sind jeweils drei Noppen 12a, 12b und 12c ringförmig um jeweils ein Belüftungsloch 7 angeordnet. Die Noppen 12a, 12b, 12c sind durch Senken seitlich voneinander beabstandet, so dass sich zwischen ihnen Strömungskanäle 13 bilden, die mit dem jeweiligen Belüftungsloch 7 und der Umgebung der Brustprothese in Strömungsverbindung stehen. Die Gruppen von ringförmig angeordneten Noppen 12a, 12b, 12c sind ebenfalls seitlich durch Senken 9 voneinander beabstandet, so dass auch zwischen den Gruppen von Noppen Strömungskanäle entstehen, durch die die Luft zu und von den Belüftungslöchern 7 strömen kann. Sowohl die Noppen 11 bei dem in Fig. 3A dargestellten Ausführungsbeispiel als auch die Noppen 12a, 12b und 12c des in Fig. 3C dargestellten Ausführungsbeispiels befinden sich nur auf der dem Oberkörper der Prothesenträgerin zugewandten Seite der textilen Stofflage. Auch bei den in den Figuren 3A und 3C dargestellten Ausführungsbeispielen sind wie bei dem Ausführungsbeispiel, das in den Figuren 1, 2 und 3 dargestellt ist, die Belüftungslöcher und die Erhebungen in einem Bereich angeordnet, der einen Abstand von einigen Millimetern von dem verschweißten Rand 6 der Stofflage hat, damit die dort befindliche Schweißnaht dicht und ununterbrochen ist.

Es versteht sich, dass die Anordnung, Form und Dichte der Belüftungslöcher und der Erhebungen innerhalb weiter Grenzen variiert werden kann.

Die in den Figuren 3A, 3B und 3C dargestellten Anordnungen, Formen und Dichten von Belüftungslöchern und Erhebungen sind nur als nicht beschränkende Beispiele zu verstehen.

## Patentansprüche

1. Textile Stofflage mit einer luftdurchlässigen Struktur, wobei die Stofflage (5) eine Vielzahl von im Abstand voneinander angeordneten Erhebungen (8; 11; 12a-12c) und eine Vielzahl von Belüftungslöchern (7) aufweist, die sich in wenigstens einigen der zwischen den Erhebungen vorhandenen Senken (9) befinden, wobei die Stofflage (5) auch in den Senken (9) zwischen den Belüftungslöchern (7) luftdurchlässig ist, **dadurch gekennzeichnet, dass** die Senken (9) an der Oberseite offene Strömungskanäle bilden, die sich von den Belüftungslöchern (7) zwischen den Erhebungen hindurch bis zum äußeren Rand der Stofflage (5) erstrecken.

2. Textile Stofflage nach Anspruch 1, **dadurch gekennzeichnet, dass** die Erhebungen (8; 11; 12a-12c) linien-, punkt und/oder flächenförmige Gebilde sind, zwischen denen sich die Senken (9) befinden, die die Strömungskanäle bilden, die mit den Belüftungslöchern (7) und der äußeren Umgebung der Stofflage (5) in Strömungsverbindung sind.

3. Textile Stofflage nach Anspruch 1 oder 2, **dadurch gekennzeichnet, dass** die Erhebungen aus länglichen Rippen (8) bestehen, die durch Senken seitlich voneinander beabstandet sind.

4. Textile Stofflage nach Anspruch 1 oder 2, **dadurch gekennzeichnet, dass** die Erhebungen aus Noppen (11) bestehen, die durch Senken seitlich voneinander beabstandet sind.

5. Textile Stofflage nach Anspruch 1 oder 2, **dadurch gekennzeichnet, dass** die Erhebungen mehrere Gruppen von jeweils mehreren ringförmig um ein Belüftungsloch (7) herum angeordneten Noppen (12a-12c) aufweisen, und die Noppen (12a-12c) der Gruppe durch Senken seitlich voneinander beabstandet sind, wobei diese Senken an der Oberseite offene Strömungskanäle (13) bilden, die mit dem jeweiligen Belüftungsloch (7)und den zwischen den Noppengruppen angeordneten Senken in Strömungsverbindung stehen.

6. Textile Stofflage nach einem der Ansprüche 1 bis 5, **dadurch gekennzeichnet, dass** die Erhebungen jeweils durch einen eine Flockbeschichtung tragenden Klebstoffauftrag auf der Stofflage (5) gebildet sind, oder dass die Erhebungen (8) durch Stricken oder Wirken gebildet sind.

7. Künstliches Brustgewebeteil zum Tragen in der Büstenschale eines Büstenschalen aufweisenden Kleidungsstücks, mit einem Körper (1), der einen weich-elastischen Kern und zwei längs einer eine geschlossene Schleife bildenden Naht fest miteinander verbundene elastische Kunststofffolien aufweist, die eine Hülle bilden, in die der Kern hohlraumfrei eingeschlossen ist, wobei der Körper (1) eine Rückseite aufweist, die mindestens eine Vertiefung (2a-2d) hat, über die sich ein Bereich einer textilen Stofflage (5) nach einem der Ansprüche 1 bis 6 erstreckt, die längs einer eine geschlossene Schleife bildenden Naht (6) mit dem Körper (1) fest verbunden ist, wobei die luftdurchlässige Struktur der textilen Stofflage (5) einen Luftaustausch zwischen der Vertiefung (2a-2d) und der äußeren Umgebung des Brustgewebeteils gestattet, wobei die textile Stofflage (5) zumindest in dem über die Vertiefung (2a-2d) sich erstreckenden Bereich die Belüftungslöcher (7) aufweist, die einen zusätzlichen Luftaustausch zwischen der Vertiefung (2a-2d) und der äußeren Umgebung des Brustgewebeteils gestatten.

8. Künstliches Brustgewebeteil nach Anspruch 7, **dadurch gekennzeichnet, dass** die Stofflage (5) zumindest im über der Vertiefung (2a-2d) (1) sich erstreckenden Bereich auf ihrer von der Rückseite des Körpers (1) abgewandten Seite die Erhebungen (8; 11; 12a-12c) aufweist, zwischen denen sich die Senken (9) befinden, wobei sich die Belüftungslöcher (7) in wenigstens einigen der Senken (9) befinden.

9. Künstliches Brustgewebeteil nach Anspruch 8, **dadurch gekennzeichnet, dass** die Erhebungen (8; 11; 12a-12c) linien-, punkt- und/oder flächenförmige Gebilde sind, zwischen denen sich die Senken (9) befinden, die an der Oberseite offene Strömungskanäle bilden, die mit den Belüftungslöchern (7) und der äußeren Umgebung des Brustgewebeteils in Strömungsverbindung sind.

10. Künstliches Brustgewebeteil nach Anspruch 8 oder 9, **dadurch gekennzeichnet, dass** die Erhebungen aus durch Senken seitlich voneinander beabstandete länglichen Rippen (8) bestehen, die sich in Längsrichtung mit oder ohne Unterbrechungen zwischen der die Stofflage (5) mit dem Körper (1) verbindenden Naht (6) erstrecken.

11. Künstliches Brustgewebeteil nach Anspruch 8 oder 9, **dadurch gekennzeichnet, dass** die Erhebungen aus durch Senken seitlich voneinander beabstandeten Noppen (11) bestehen, die im Bereich innerhalb der die Stofflage (5) mit dem Körper (1) verbindenden Naht (6) verteilt angeordnet sind.

12. Künstliches Brustgewebeteil nach einem der Ansprüche 7 bis 11, wobei die textile Stofflage (5) durch Schweißen mit dem Körper längs der die Stofflage (5) mit dem Körper verbindenden Naht (6) fest verbunden ist, **dadurch gekennzeichnet, dass** sich der die Belüftungslöcher (7) aufweisende Bereich der textilen Stofflage (5) nicht in die Schweißnaht (6) erstreckt.

13. Künstliches Brustgewebeteil nach einem der Ansprüche 7 bis 12, wobei die beiden Kunststofffolien und die textile Stofflage längs einer eine geschlossene Schleife bildenden, gemeinsamen Naht (4) miteinander verschweißt sind, **dadurch gekennzeichnet, dass** der die Belüftungslöcher (7) und die Erhebungen (8; 11; 12a-12c) aufweisende Bereich der textilen Stofflage (5) sich nicht in die die Kunststofffolien und die Stofflage (5) miteinander verbindenden gemeinsamen Schweißnaht (4, 6) erstreckt.

14. Künstliches Brustgewebeteil nach einem der Ansprüche 7 bis 13, **dadurch gekennzeichnet, dass** der Körper (1) auf seiner Rückseite wenigstens zwei durch eine Rippe (3a-3b) voneinander getrennte Vertiefungen (2a-2d) hat.

15. Künstliches Brustgewebeteil nach einem der Ansprüche 7 bis 14, **dadurch gekennzeichnet, dass** es eine Brustprothese für brustamputierte Frauen ist, oder dass es ein Brustgewebeausgleichsteil für Frauen mit teilamputierter Brust ist, oder dass es ein die natürliche Brust stützendes und/oder vergrößerndes Formteil ist.

## Claims

1. A textile fabric layer having an air-permeable structure, with the fabric layer (5) having a multiplicity of spaced apart elevations (8; 11; 12a-12c) and a multiplicity of airing holes (7) which are located in at least some of the depressions (9) present between the elevations, with the fabric layer (5) also being air-permeable in the depressions (9) between the airing holes (7), **characterized in that** the depressions (9) form flow channels open on the upper side which extend from the airing holes (7) through between the elevations as far as the outer edge of the fabric layer (5).

2. The textile fabric layer according to claim 1, **characterized in that** the elevations (8; 11; 12a-12c) are linear, punctiform and/or areal entities between which are located the depressions (9) which form the flow channels which are in flow communication with the airing holes (7) and the outer surroundings of the fabric layer (5).

3. The textile fabric layer according to claim 1 or 2, **characterized in that** the elevations consist of elongate ribs (8) which are spaced apart laterally by depressions.

4. The textile fabric layer according to claim 1 or 2, **characterized in that** the elevations consist of nubs (11) which are spaced apart laterally by depressions.

5. The textile fabric layer according to claim 1 or 2, **characterized in that** the elevations have a plurality of groups each with a plurality of nubs (12a-12c) arranged annularly around an airing hole (7), and the nubs (12a-12c) of the group are spaced apart laterally by depressions, with said depressions forming flow channels (13) open on the upper side which are in flow communication with the respective airing hole (7) and the depressions arranged between the groups of nubs.

6. The textile fabric layer according to any of claims 1 to 5, **characterized in that** the elevations are respectively formed by an application of adhesive bearing a flocked coating, on the fabric layer (5), or that the elevations (8) are formed by knitting.

7. An artificial breast-tissue piece to be worn in the breast cup of a garment having breast cups, having a body (1) which has a soft-elastic core and two elastic synthetic foils firmly interconnected along a seam forming a closed loop, said foils forming a sheath in which the core is enclosed without any cavity, with the body (1) having a back side which has at least one recess (2a-2d) over which a region of a textile fabric layer (5) according to any of claims 1 to 6 extends, which is firmly connected to the body (1) along a seam (6) forming a closed loop, with the air-permeable structure of the textile fabric layer (5) permitting an air exchange between the recess (2a-2d) and the outer surroundings of the breast-tissue piece, with the textile fabric layer (5) having at least in the region extending over the recess (2a-2d) the airing holes (7) which permit an additional air exchange between the recess (2a-2d) and the outer surroundings of the breast-tissue piece.

8. The artificial breast-tissue piece according to claim 7, **characterized in that** the fabric layer (5) has at least in the region extending over the recess (2a-2d) (1), on its side facing away from the back side of the body (1), the elevations (8; 11; 12a-12c) between which the depressions (9) are located, with the airing holes (7) being located in at least some of the depressions (9).

9. The artificial breast-tissue piece according to claim 8, **characterized in that** the elevations (8; 11; 12a-12c) are linear, punctiform and/or areal entities between which are located the depressions (9) which form flow channels open on the upper side which are in flow communication with the airing holes (7) and the outer surroundings of the breast-tissue piece.

10. The artificial breast-tissue piece according to claim 8 or 9, **characterized in that** the elevations consist of elongate ribs (8) spaced apart laterally by depressions, said ribs extending in the longitudinal direction with or without interruptions between the seam (6) connecting the fabric layer (5) to the body (1).

11. The artificial breast-tissue piece according to claim 8 or 9, **characterized in that** the elevations consist of nubs (11) spaced apart laterally by depressions, said nubs being arranged in a distributed manner in the region within the seam (6) connecting the fabric layer (5) to the body (1).

12. The artificial breast-tissue piece according to any of claims 7 to 11, wherein the textile fabric layer (5) is firmly connected by welding to the body along the seam (6) connecting the fabric layer (5) to the body, **characterized in that** the region of the textile fabric layer (5) having the airing holes (7) does not extend into the weld seam (6).

13. The artificial breast-tissue piece according to any of claims 7 to 12, wherein the two synthetic foils and the textile fabric layer are welded together along a common seam (4) forming a closed loop, **characterized in that** the region of the textile fabric layer (5) having the airing holes (7) and the elevations (8; 11; 12a-12c) does not extend into the common weld seam (4, 6) interconnecting the synthetic foils and the fabric layer (5).

14. The artificial breast-tissue piece according to any of claims 7 to 13, **characterized in that** the body (1) has on its back side at least two recesses (2a-2d) mutually separated by a rib (3a-3b).

15. The artificial breast-tissue piece according to any of claims 7 to 14, **characterized in that** it is a breast prosthesis for mastectomized women, or that it is a breast-tissue compensation piece for women with a partial mastectomy, or that it is a formed piece supporting and/or enlarging the natural breast.

## Revendications

1. Couche de tissu textile avec une structure perméable à l'air, la couche de tissu (5) comprenant une pluralité de saillies (8 ; 11 ; 12a-12c) distantes entre elles et une pluralité de trous d'aération (7) qui se trouvent dans au moins certains des creux (9) existant entre les saillies, la couche de tissu (5) étant perméable à l'air également dans les creux (9) entre les trous d'aération (7), **caractérisée en ce que** les creux (9) forment, en haut, des canaux d'écoulement ouverts qui s'étendent à partir des trous d'aération (7) entre les saillies jusqu'au bord extérieur de la couche de tissu (5).

2. Couche de tissu textile selon la revendication 1, **caractérisée en ce que** les saillies (8 ; 11 ; 12a-12c) sont des structures linéaires, ponctuelles et/ou planes entre lesquelles se trouvent les creux (9) qui forment les canaux d'écoulement en liaison fluidique avec les trous d'aération (7) et l'environnement extérieur de la couche de tissu (5).

3. Couche de tissu textile selon la revendication 1 ou 2, **caractérisée en ce que** les saillies sont constituées de nervures allongées (8) qui sont écartées entre elles latéralement par des creux.

4. Couche de tissu textile selon la revendication 1 ou 2, **caractérisée en ce que** les saillies sont constituées de picots (11) qui sont écartés entre eux latéralement par des creux.

5. Couche de tissu textile selon la revendication 1 ou 2, **caractérisée en ce que** les saillies comprennent plusieurs groupes de picots (12a-12c) disposés autour d'un trou d'aération (7) de manière annulaire et les picots (12a-12c) du groupe sont écartées entre eux latéralement par des creux, ces creux formant, en haut, des canaux d'écoulement ouverts (13) en liaison fluidique avec le trou d'aération (7) correspondant et les creux disposés entre les groupes de picots.

6. Couche de tissu textile selon l'une des revendications 1 à 5, **caractérisée en ce que** les saillies sont formées chacune d'une application de colle comportant un revêtement par flocage sur la couche de tissu (5) ou **en ce que** les saillies (8) sont formés par tricotage ou filage.

7. Pièce de tissu de poitrine artificielle destiné à être portée dans la coque de poitrine d'un vêtement comprenant une coque de poitrine, avec un corps (1), qui comprend un noyau souple élastique et deux films de matière plastique reliés entre eux le long d'une couture formant une boucle fermée, qui forment une enveloppe dans laquelle le noyau est intégré sans espace libre, le corps (1) comprenant un côté arrière qui comprend au moins une cavité (2a-2d) au-dessus de laquelle s'étend une zone d'une couche de tissu textile (5) selon l'une des revendications 1 à 6, qui est reliée avec le corps (1) le long d'une couture (6) formant une boucle fermée, la structure perméable à l'air de la couche de tissu textile (5) permettant un échange d'air entre la cavité (2a-2d) et l'environnement extérieur de la pièce de tissu de poitrine, la couche de tissu textile (5) comprenant, au moins dans la zone s'étendant au-dessus de la cavité (2a-2d), les trous d'aération (7) qui permettent un échange d'air supplémentaire entre la cavité (2a-2d) et l'environnement extérieur de la pièce de tissu de poitrine.

8. Pièce de tissu de poitrine artificielle selon la revendication 7, **caractérisée en ce que** la couche de tissu (5) comprend, au moins dans la zone s'étendant au-dessus de la cavité (2a-2d) (1), sur son côté opposé au côté arrière du corps (1), les saillies (8; 11 ; 12a-12c) entre lesquelles se trouvent les creux (9), les trous d'aération (7) se trouvant dans au moins certains des creux (9).

9. Pièce de tissu de poitrine artificielle selon la revendication 8, **caractérisée en ce que** les saillies (8 ; 11 ; 12a-12c) sont des structures linéaires, ponctuelles et/ou planes entre lesquelles se trouvent les creux (9), qui forment, en haut, des canaux d'écoulement ouverts en liaison fluidique avec les trous d'aération (7) et l'environnement extérieur de la pièce de tissu de poitrine.

10. Pièce de tissu de poitrine artificielle selon les revendications 8 ou 9, **caractérisée en ce que** les saillies sont constituées de nervures (8) allongées écartées latéralement entre elles par des creux, qui s'étendent dans la direction longitudinale avec ou sans interruption entre la couture (6) reliant la couche de tissu (5) avec le corps (1).

11. Pièce de tissu de poitrine artificielle selon les revendications 8 ou 9, **caractérisée en ce que** les saillies sont constituées de picots (11) écartés latéralement entre eux par des creux, qui sont disposés de manière répartie dans la zone à l'intérieur de la couture (6) reliant la couche de tissu (5) avec le corps (1).

12. Pièce de tissu de poitrine artificielle selon l'une des revendications 7 à 11, la couche de tissu textile (5) étant reliée fermement par soudure avec le corps le long de la couture (6) reliant la couche de tissu (5) avec le corps, **caractérisée en ce que** la zone de la couche de tissu textile (5) comprenant les trous d'aération (7) ne s'étend pas dans le cordon de soudure (6).

13. Pièce de tissu de poitrine artificielle selon l'une des revendications 7 à 12, les deux films de matière plastique et la couche de tissu textile étant soudés entre eux le long d'une couture (4) commune formant une boucle fermée, **caractérisée en ce que** la zone de la couche de tissu textile (5) comprenant les trous d'aération (7) et les saillies (8; 11 ; 12a-12c) ne s'étend pas dans le cordon de soudure (4, 6) commun reliant entre eux les films de matière plastique et la couche de tissu (5).

14. Pièce de tissu de poitrine artificielle selon l'une des revendications 7 à 13, **caractérisée en ce que** le corps (1) comprend, sur son côté arrière, au moins deux cavités (2a-2d) séparés entre eux par une nervure (3a-3b).

15. Pièce de tissu de poitrine artificielle selon l'une des revendications 7 à 14, **caractérisée en ce qu'**il s'agit d'une prothèse de poitrine pour des femmes ayant subi une mammectomie ou **en ce qu'**il s'agit d'une pièce pour compensation de poitrine pour des femmes ayant subi une mammectomie partielle ou **en ce qu'**il s'agit d'une pièce moulée soutenant et/ou grossissant une poitrine naturelle.
